Europäisches Patentamt

European Patent Office

Office européen des brevets

(19)

(11) Numéro de publication : **0 442 821 B1**

(12) **FASCICULE DE BREVET EUROPEEN**

(45) Date de publication du fascicule du brevet :
**23.06.93 Bulletin 93/25**

(51) Int. Cl.⁵ : **A61K 7/06**

(21) Numéro de dépôt : **91400394.2**

(22) Date de dépôt : **15.02.91**

(54) **Laque aérosol capillaire contenant un tétrapolymère d'acide acrylique, de N,N-diméthylacrylamide, de N-tertiobutylacrylamide et de méthacrylate d'éthyle et un gaz propulseur non halogéné.**

(30) Priorité : **16.02.90 FR 9001917**

(43) Date de publication de la demande :
**21.08.91 Bulletin 91/34**

(45) Mention de la délivrance du brevet :
**23.06.93 Bulletin 93/25**

(84) Etats contractants désignés :
**AT BE CH DE DK ES FR GB GR IT LI NL SE**

(56) Documents cités :
**EP-A- 0 062 002
DE-A- 3 518 847
FR-A- 2 424 738
US-A- 3 927 199**

(73) Titulaire : **L'OREAL
14, Rue Royale
F-75008 Paris (FR)**

(72) Inventeur : **Mondet, Jean
34, rue Daniel Fery
F-93700 Drancy (FR)**
Inventeur : **Papantoniou, Christos
17, rue des Basserons
F-95160 Montmorency (FR)**
Inventeur : **Cheneble, Jean-Charles
76, rue Roger Salengro
F-93140 Bondy (FR)**
Inventeur : **Mahieu, Claude
90, avenue de Villiers
F-75017 Paris (FR)**

(74) Mandataire : **Stalla-Bourdillon, Bernard et al
Cabinet Nony 29, rue Cambacérès
F-75008 Paris (FR)**

**Description**

La présente invention a pour objet une composition cosmétique sous forme d'une laque aérosol pour la fixation des cheveux contenant, en tant que substance filmogène, un tétrapolymère d'acide acrylique, de N, N-diméthylacrylamide, de N-tertiobutylacrylamide et de méthacrylate d'éthyle, le gaz propulseur étant du type non halogéné.

Pour des raisons écologiques, la tendance actuelle consiste à remplacer systématiquement, dans les bombes aérosols pour cheveux, les gaz propulseurs halogénés du type Fréon par des hydrocarbures, en particulier par du propane ou du butane ou leurs mélanges. Toutefois une telle subtitution ne peut se faire sans modifier profondément les formulations non seulement en ce qui concerne les rapports entre les ingrédients mais également en ce qui concerne la nature même de ceux-ci et tout particulièrement les substances filmogènes.

Il est en effet bien connu qu'une laque aérosol pour la fixation des cheveux doit satisfaire à un certain nombre de critères. Parmi ceux-ci on doit tout particulièrement mentionner les propriétés de fixation de la chevelure en atmosphère à forte teneur en humidité sans constater un effet de poissage de la chevelure.

Il convient en effet qu'entre deux shampooings les cheveux conservent de bonnes propriétés cosmétiques au toucher, ne poissent pas ni ne graissent et se démêlent facilement sans se coller les uns aux autres, surtout lorsque les shampooings sont espacés d'au moins une semaine

Par ailleurs, il importe que la substance filmogène puisse être facilement éliminée lors du lavage de la chevelure. Enfin, il convient que la chevelure après application de la laque aérosol présente un aspect naturel et que l'on puisse peigner les cheveux sans que l'on constate un effet de poudrage.

Toutes ces propriétés ont pu être mises en évidence pour certains polymères qui ont été utilisés jusqu'ici pour la réalisation de laques aérosols dont l'agent propulseur était essentiellement un mélange d'hydrocarbures halogénés, notamment les mélanges de monofluorotrichlorométhane et de dichlorodifluorométhane.

Des polymères tels que ceux par exemple à base d'acétate de vinyle et d'acide crotonique se sont toutefois avérés être totalement inutilisables en présence de gaz propulseurs non halogénés.

Les travaux récents ont donc essentiellement portés sur le choix de nouveaux polymères susceptibles non seulement d'être compatibles avec des gaz propulseurs non halogénés mais de présenter également d'excellentes propriétés cosmétiques.

On peut à cet égard citer le Brevet Européen n° 0062.002 qui décrit une composition pour la fixation des cheveux contenant, en tant que substance filmogène, un terpolymère résultant de la copolymérisation :

(a) de 40 à 60% en poids d'un N-alkylacrylamide ou d'un N-alkylméthacrylamide ayant de 1 à 4 atomes de carbone dans la partie alkyle, avec

(b) de 35 à 50% en poids d'un ester d'alkyle en $C_1$-$C_4$ ou d'un ester d'hydroxyalkyle en $C_1$-$C_4$ de l'acide acrylique ou de l'acide méthacrylique, et

(c) de 3 à 11% en poids d'acide acrylique ou d'acide méthacrylique, par rapport au total du mélange de monomères et une proportion d'au moins 50% des groupes carboxyles présents dans le terpolymère étant neutralisés par une base organique inférieure choisie parmi l'amino-2-méthyl-2-propanol, l'amino-2-méthyl-2-propanediol-1,3, la triéthanolamine et la tri-isopropanolamine, le gaz propulseur étant un hydrocarbure non halogéné tel que le propane ou le butane ou un mélange de ceux-ci.

Si le choix des monomères de ce terpolymère a permis une bonne compatibilité avec les hydrocarbures non halogénés, on a toutefois constaté que certaines des propriétés cosmétiques des laques aérosols n'étaient pas totalement satisfaites.

En particulier, tantôt on observe une insolubilité de la laque par les shampooings, tantôt on observe un effet de poissage important des cheveux après une période d'environ 7 jours après l'application de la laque.

Après différentes études sur un très grand nombre de copolymères, on vient maintenant de constater de façon tout-à-fait surprenante et innatendue que d'excellentes propriétés cosmétiques ainsi qu'une très bonne compatibilité avec les gaz propulseurs non halogénés pouvaient être obtenues grâce à un tétrapolymère contenant des unités d'acide acrylique, de N,N-diméthylacrylamide, de N-tertiobutylacrylamide et de méthacrylate d'éthyle.

Ces études ont non seulement permis de mettre en évidence l'importance du choix des monomères mais également le caractère critique des proportions de chacun de ceux-ci.

La présente invention a donc pour objet une composition cosmétique sous forme d'une laque aérosol pour la fixation des cheveux, contenant dans un solvant, en tant que substance filmogène, un tétrapolymère résultant de la copolymérisation :

(a) de 5 à 10% en poids d'acide acrylique (AA) et de préférence de 5 à 8% en poids,

(b) de 20 à 52% en poids de N, N-diméthylacrylamide (DMA) et de préférence de 25 à 45% en poids,

(c) de 3 à 28% en poids de N-tertiobutylacrylamide (Nt BA) et de préférence de 5 à 25% en poids, et

(d) de 20 à 45% en poids de méthacrylate d'éthyle (MAE) et de préférence de 25 à 40% en poids, les fonctions acides carboxyliques dudit tétrapolymère étant neutralisées, partiellement ou totalement, à l'aide d'une base minérale ou organique, et en tant que gaz propulseur, au moins un composé non halogéné.

Les bonnes propriétés des laques aérosols selon l'invention sont dues essentiellement aux choix des monomères du tétrapolymère et à leurs proportions respectives qui ne varient que dans une gamme relativement étroite et critique.

Le taux de neutralisation des fonctions carboxyliques du tétrapolymère est d'au moins 50% et de préférence compris entre 70 et 100%.

Parmi les bases minérales susceptibles d'être utilisées on peut notamment citer la soude, la potasse ou l'ammoniaque et parmi les bases organiques les amines primaires, secondaires ou tertiaires, les alcanolamines telles que la triéthanolamine ou la tri-isopropanolamine, les hydroxy-amines tels que l'amino-2-méthyl-2-propanol (AMP) et l'amino-2-méthyl-2-propanediol-1,3 (AMPD).

Le tétrapolymère des laques aérosols selon l'invention doit de préférence avoir un poids moléculaire tel que sa viscosité absolue soit comprise entre 2 et 5mPa.s(cP) (mesurée en solution dans le DMF à 5% et à 34,6°C) et de préférence comprise entre 2,3 et 4,5mPa.s(cP).

Dans les laques aérosols selon l'invention le tétrapolymère est généralement présent, en solution dans un solvant organique, à une concentration comprise entre 0,5 et 5% en poids et de préférence entre 1 et 4% en poids par rapport au poids total de la composition. Le solvant est de préférence un alcool inférieur présent à une concentration comprise entre 40 et 60% en poids et parmi ceux-ci on peut notamment mentionner l'éthanol, l'isopropanol et leurs mélanges. La proportion du gaz propulseur non halogéné est généralement comprise entre 40 et 65% en poids et de préférence entre 45 et 60%.

Comme gaz propulseur de la laque aérosol selon l'invention on utilise de préférence un hydrocarbure non halogéné tel que le propane, le n-butane, l'isobutane ou leurs mélanges, en particulier les mélanges de propane-butanes dans un rapport de 1 : 10 à 1 : 2, ou encore le diméthyléther ou un mélange de ces gaz propulseurs.

Bien entendu les laques aérosols selon l'invention peuvent également contenir d'autres ingrédients conventionnels pour ce type de composition tels que des agents plastifiants, des agents adoucissants, des parfums, des huiles, des lubrifiants, de la lanoline, des silicones, des filtres solaires ainsi que des colorants.

Selon l'invention il est souhaitable que dans la bombe aérosol la pression de vapeur soit comprise entre environ 2,5 et 5 bars à 25°C.

Le tétrapolymère de la laque aérosol selon l'invention peut être obtenue selon les techniques conventionnelles de polymérisation c'est-à-dire en masse, en solution, en suspension, en dispersion ou en émulsion sans que les caractéristiques essentielles du tétrapolymère obtenu en soient affectées.

La réaction de polymérisation peut être initiée à l'aide de tous catalyseurs conventionnels tels que l'azobisisobutyronitrile ou des composés organiques peroxydés comme le peroxyde de lauroyle ou le peroxyde de benzoyle.

En vue de régler le poids-moléculaire, il peut être souhaitable d'utiliser des agents de transfert des chaînes tels que des thiols par exemple le mercaptoéthanol ou le dodécanethiol ou encore des accélérateurs qui augmentent la vitesse de réaction.

Selon un mode de réalisation préféré la polymérisation est effectuée en solution, de préférence dans l'éthanol, et en fin de polymérisation, le tétrapolymère est isolé par précipitation notamment dans de l'éther de pétrole.

Selon cette forme de réalisation la polymérisation en solution est généralement conduite à une température comprise entre 40 et 90°C et de préférence entre 45 et 80°C.

On va maintenant donner à titre d'illustration plusieurs exemples de préparation des tétrapolymères ainsi que plusieurs exemples de laques aérosols les contenant.

EXEMPLE 1 : Préparation du tétrapolymère "acide acrylique (AA) 6%/N, N-diméthylacrylamide (DMA) 40%/N-tertio-butylacrylamide (Nt BA) 19%/métacrylate d'éthyle (MAE) 35%".

Dans un ballon, sous agitation et sous azote, on a introduit 12g d'acide acrylique, 80g de N, N-diméthylacrylamide, 38g de N-tertiobutylacrylamide et 70g de méthacrylate d'éthyle.

On ajoute alors 200g d'éthanol et 2g d'azobisisobutyronitrile en tant que catalyseur.

Le mélange est alors chauffé sous agitation pendant 30 minutes à 74°C puis on maintient cette température pendant environ 8 heures.

Après avoir laissé refroidir, on dilue avec 600g d'acétate d'éthyle et on précipite le tétrapolymère obtenu dans 10 litres d'éther de pétrole.

Après purification et s'échage sous vide, le tétrapolymère attendu est obtenu avec un rendement de 98%.

Viscosité ($\eta$) : 3,40 cP (mesurée en solution à 5% dans le DMF et à 34,6°C)

Indice d'acide (IA) : 47

Température de transition vitreuse (Tg) : 108 (mesurée en DSC sur un appareil PERKIN-ELMER DSC 4 calibré à la température de fusion de l'indium, la vitesse de chauffe étant de 20°C/min.).

Selon le même mode opératoire que décrit ci-dessus on a également préparé les tétrapolymères rassemblés dans le Tableau I suivant :

TABLEAU I

| EXEMPLES | AA % | DMA % | Nt BA % | MAE % | Rdt % | $\eta$ | IA | Tg°C |
|---|---|---|---|---|---|---|---|---|
| 2 | 10 | 36 | 19 | 35 | 98 | 3,20 | 76 | 117 |
| 3 | 9 | 47 | 19 | 25 | 98 | 3,26 | 68 | 124 |
| 4 | 6 | 37 | 13 | 44 | 98 | 3,02 | 43 | 103 |
| 5 | 8 | 32 | 25 | 35 | 99 | 3,04 | 57 | 112 |
| 6 | 7 | 29 | 19 | 45 | 99 | 2,92 | 55 | 105 |
| 7 | 5 | 47 | 13 | 35 | 100 | 2,84 | 35 | 108 |
| 8 | 8 | 45 | 5 | 42 | 100 | 3,10 | 61 | 101 |
| 9 | 8 | 40 | 10 | 42 | 99 | 3,03 | 61 | 102 |
| 10 | 8 | 30 | 20 | 42 | 100 | 3,00 | 61,6 | 104 |
| 11 | 6 | 43 | 25 | 26 | 100 | 3,14 | 44 | 122 |
| 12 | 5 | 51 | 19 | 25 | 94 | 3,12 | 37 | 117 |
| 13 | 8 | 25 | 25 | 42 | 93 | 3,05 | 63 | 106 |

Exemples de laques aérosols

Exemple A :

On prépare une laque aérosol pour cheveux en conditionnant dans un récipient aérosol 2g du tétrapolymère préparé selon l'exemple 1, neutralisé à 100% (d'après l'indice d'acide) par de l'AMP, et 40g d'éthanol anhydre. On introduit ensuite selon les techniques conventionnelles 58g d'un mélange propulseur constitué de butane : 55, propane : 10 et isobutane : 35 et on procède à la fixation de la valve et à la fermeture hermétique du récipient (pression : 4 bars).

Par pulvérisation de la laque sur des cheveux naturels ou sensibilisés on constate que celle-ci a un bon pouvoir laquant, une absence de poissage des cheveux au toucher même après plusieurs jours, ceux-ci se démêlant facilement et ne provoquent qu'un léger effet de poudrage.

Après plusieurs jours sans shampooing ces propriétés sont maintenues. On note en effet une absence de regraissage et de poissage et un état des cheveux excellent.

Après shampooing la substance filmogène est parfaitement éliminée, les cheveux présentant de très bonnes propriétés de démêlage, de douceur et un toucher agréable.

Exemple B :

On prépare une laque aérosol pour cheveux en conditionnant dans un récipient aérosol 2g du tétrapolymère préparé selon l'exemple 10, neutralisé à 100% (d'après l'indice d'acide) par de l'AMP, et 43g d'éthanol anhydre. On introduit ensuite 55g d'un mélange propulseur constitué de butane : 55, propane : 10 et isobutane : 35 et on procède à la fixation de la valve et à la fermeture hermétique du récipient (pression : 4,3 bars).

Exemple C :

On prépare une laque aérosol pour cheveux en conditionnant dans un récipient aérosol 2g du tétrapolymère préparé selon l'exemple 11, neutralisé à 100% (d'après l'indice d'acide) par de l'AMP, et 43g d'éthanol anhydre. On introduit ensuite 55g d'un mélange propulseur constitué de butane : 55, propane : 10 et isobutane : 35 et on procède à la fixation de la valve et à la fermeture hermétique du récipient (pression : 4,5 bars).

Pour les laques des exemples B et C ci-dessus on a constaté à l'application sur des cheveux naturels ou sensibilisés les mêmes bonnes propriétés que celles obtenues pour la laque aérosol de l'exemple A.

ETUDE COMPARATIVE

Afin de mettre en évidence l'importance non seulement du choix des monomères du tétrapolymère mais également leurs proportions relatives sur les propriétés cosmétiques des laques aérosols selon l'invention, on a soumis les polymères rassemblés dans le Tableau II+ d'une part à un test de poissage et d'autre part à un test de solubilité de la substance filmogène.

Ces tests ont été réalisés dans les conditions suivantes :

1) Test de poissage :

Dans un moule revêtu de "Teflon"* de 25 cm², on introduit 5g d'une solution éthanolique à 10% en poids du polymère neutralisé à 100% par l'AMP, puis le solvant est évaporé 24 heures à température ambiante et le film est décollé du moule. Le poissage sur les deux faces du film est estimé au toucher par un panel de 5 personnes.

2) Test de solubilité :

Dans un cristallisoire de 55 mm de diamètre, on introduit 2g d'une solution éthalonique à 10% en poids du polymère neutralisé à 100% par l'AMP, puis le solvant est évaporé 24 heures à température ambiante. Sur le film sec, on verse 20g d'une solution aqueuse à 1,4% de lauryléther sulfate de sodium. La dissolution doit être complète sous agitation magnétique en moins de 15 min. en donnant une solution limpide.

TABLEAU II

| Polymères / Monomères | Exemple Ref 1' | Exemple 8 | Exemple 9 | Exemple 10 | Exemple 13 | Exemple Ref 2' | Exemple Ref 3' |
|---|---|---|---|---|---|---|---|
| AA | 8 | 8 | 8 | 8 | 8 | 8 | 8 |
| DMA | 50 | 45 | 40 | 30 | 25 | 20 | 0 |
| Nt BA | 0 | 5 | 10 | 20 | 25 | 30 | 50 |
| MAE | 42 | 42 | 42 | 42 | 42 | 42 | 42 |
| Propriétés cosmétiques * | P / S | AP / S | AP / S | AP / S | AP / S | AP / IS | AP / IS |

* P  = Poissage

AP = Absence de poissage

S  = Soluble dans shampooing

IS = Insoluble dans shampooing

* marque déposée

Comme on peut le constater d'après le Tableau II seuls les tétrapolymères des exemples 8, 9, 10 et 13, ayant des pourcentages en poids des 4 monomères compris dans les proportions indiquées précédemment, présentent des propriétés cosmétiquement satisfaisantes.

Dans les mêmes conditions que ci-dessus, les tétrapolymères des exemples 1 à 7, 11 et 12 ont été testés et se sont également avérés ne pas présenter de poissage et être solubles dans les shampooings.

**Revendications**

1. Composition cosmétique sous forme d'une laque aérosol pour la fixation des cheveux, caractérisée par le fait qu'elle contient, dans un solvant, en tant que substance filmogène, un tétrapolymère résultant de la copolymérisation :
   (a) de 5 à 10% en poids d'acide acrylique,
   (b) de 20 à 52% en poids de N, N-diméthylacrylamide,
   (c) de 3 à 28% en poids de N-tertiobutylacrylamide, et
   (d) de 20 à 45% en poids de méthacrylate d'éthyle,
   les fonctions acides carboxyliques dudit tétrapolymère étant neutralisées, partiellement ou totalement, à l'aide d'une base minérale ou organique et en tant que gaz propulseur, au moins un composé non halogéné.

2. Composition cosmétique selon la revendication 1, caractérisée par le fait que le tétrapolymère résulte de la copolymérisation :
   (a) de 5 à 8% en poids d'acide acrylique,
   (b) de 25 à 45% en poids de N, N-diméthylacrylamide,
   (c) de 5 à 25% en poids de N-tertiobutylacrylamide, et
   (d) de 25 à 40% en poids de méthacrylate d'éthyle.

3. Composition cosmétique selon la revendication 1 ou 2 caractérisée par le fait que le tétrapolymère présente une viscosité absolue comprise entre 2 et 5 mPa.s(cP) mesurée en solution dans le DMF à 5% et à 34,6°C.

4. Composition cosmétique selon l'une quelconque des revendications précédentes caractérisée par le fait que les fonctions acides carboxyliques du tétrapolymère sont neutralisées à un taux d'au moins 50% et de préférence compris entre 70 et 100%.

5. Composition selon l'une quelconque des revendications 1 à 4 caractérisée par le fait que les fonctions acides carboxyliques sont neutralisées à l'aide d'une base minérale choisie parmi la soude, la potasse et l'ammoniaque.

6. Composition cosmétique selon l'une quelconque des revendications 1 à 4 caractérisée par le fait que les fonctions acides carboxyliques sont neutralisées à l'aide d'une base organique choisie parmi la triéthanolamine, la tri-isopropanolamine, l'amino-2-méthyl-2-propanol et l'amino-2-méthyl-2-propanediol-1,3.

7. Composition cosmétique selon l'une quelconque des revendications précédentes caractérisée par le fait que le tétrapolymère est présent à une concentration comprise entre 0,5 et 5% et de préférence entre 1 et 4% en poids par rapport au poids total de la composition.

8. Composition cosmétique selon l'une quelconque des revendications précédentes caractérisée par le fait que le gaz propulseur est présent en une proportion comprise entre 40 et 65% en poids.

9. Composition cosmétique selon l'une quelconque des revendications précédentes caractérisée par le fait que le gaz propulseur non halogéné est un hydrocarbure non halogéné pris dans le groupe constitué par le propane, le n-butane, l'isobutane et leurs mélanges.

10. Composition cosmétique selon la revendication 9 caractérisée par le fait que le gaz propulseur non halogéné est un mélange propane-butanes dans un rapport de 1 : 10 à 1 : 2.

11. Composition cosmétique selon l'une quelconque des revendications 1 à 9 caractérisée par le fait que le gaz propulseur non halogéné est le diméthyléther ou son mélange avec au moins un hydrocarbure non halogéné.

**12.** Composition cosmétique selon l'une quelconque des revendications précédentes caractérisée par le fait que le solvant est présent en une proportion comprise entre 40 et 60% en poids par rapport au poids total de la composition.

**13.** Composition cosmétique selon l'une quelconque des revendications précédentes caractérisée par le fait que le solvant est l'éthanol, l'isopropanol ou leurs mélanges.

## Patentansprüche

**1.** Kosmetische Zubereitung in Form eines Aerosol-Lacks zur Fixierung der Haare, dadurch gekennzeichnet, daß sie in einem Lösungsmittel als filmbildende Substanz ein Tetrapolymer enthält, das aus der Copolymerisation von

(a) 5 bis 10 Gew.-% Acrylsäure,
(b) 20 bis 52 Gew.-% N,N-Dimethylacrylamid,
(c) 3 bis 28 Gew.-% N-tert-Butylacrylamid, und
(d) 20 bis 45 Gew.-% Ethylmethacrylat

resultiert, wobei die Carbonsäurefunktionen des Tetrapolymers teilweise oder vollständig mittels einer mineralischen oder organischen Base neutralisiert sind, und als Treibgas wenigstens eine nichthalogenierte Verbindung enthält.

**2.** Kosmetische Zubereitung nach Anspruch 1, dadurch gekennzeichnet, daß das Tetrapolymer aus der Copolymerisation von

(a) 5 bis 8 Gew.-% Acrylsäure,
(b) 25 bis 45 Gew.-% N,N-Dimethylacrylamid,
(c) 5 bis 25 Gew.-% N-tert-Butylacrylamid, und
(d) 25 bis 40 Gew.-% Ethylmethacrylat

resultiert.

**3.** Kosmetische Zubereitung nach Anspruch 1 oder 2, dadurch gekennzeichnet, daß das Tetrapolymer eine absolute Viskosität zwischen 2 und 5 mPas (cP) aufweist, gemessen in einer 5 %-igen Lösung in DMF bei 34,6°C.

**4.** Kosmetische Zubereitung nach einem der vorangehenden Ansprüche, dadurch gekennzeichnet, daß die Carbonsäurefunktionen des Tetrapolymers zu wenigstens 50 % und mit Vorzug zwischen 70 und 100 % neutralisiert sind.

**5.** Zubereitung nach einem der Ansprüche 1 bis 4, dadurch gekennzeichnet, daß die Carbonsäurefunktionen mittels einer mineralischen Base neutralisiert sind, die gewählt ist aus Natriumcarbonat (Soda), Kaliumcarbonat (Pottasche) und Ammoniak.

**6.** Kosmetische Zubereitung nach einem der Ansprüche 1 bis 4, dadurch gekennzeichnet, daß die Carbonsäurefunktionen mittels einer organischen Base neutralisiert sind, die gewählt ist aus Triethanolamin, Triisopropanolamin, Amino-2-methyl-2-propanol und Amino-2-methyl-2-propan-1,3-diol.

**7.** Kosmetische Zubereitung nach einem der vorangehenden Ansprüche, dadurch gekennzeichnet, daß das Tetrapolymer in einer Konzentration zwischen 0,5 und 5 Gew.-%, mit Vorzug zwischen 1 und 4 Gew.-% zugegen ist, bezogen auf das Gesamtgewicht der Zubereitung.

**8.** Kosmetische Zubereitung nach einem der vorangehenden Ansprüche, dadurch gekennzeichnet, daß das Treibgas in einer Menge zwischen 40 und 65 Gew.-% zugegen ist.

**9.** Kosmetische Zubereitung nach einem der vorangehenden Ansprüche, dadurch gekennzeichnet, daß das nichthalogenierte Treibgas ein nichthalogenierter Kohlenwasserstoff aus der aus Propan, n-Butan, Isobutan und ihren Mischungen bestehenden Gruppe ist.

**10.** Kosmetische Zubereitung nach Anspruch 9, dadurch gekennzeichnet, daß das nichthalogenierte Treibgas eine Mischung von Propan und Butanen im Verhältnis 1 : 10 bis 1 : 2 ist.

11. Kosmetische Zubereitung nach einem der Ansprüche 1 bis 9, dadurch gekennzeichnet, daß das nichthalogenierte Treibgas Dimethylether oder eine Mischung davon mit wenigstens einem nichthalogenierten Kohlenwasserstoff ist.

12. Kosmetische Zubereitung nach einem der vorangehenden Ansprüche, dadurch gekennzeichnet, daß das Lösungsmittel in einer Menge zwischen 40 und 60 Gew.-% zugegen ist, bezogen auf das Gesamtgewicht der Zubereitung.

13. Kosmetische Zubereitung nach einem der vorangehenden Ansprüche, dadurch gekennzeichnet, daß das Lösungsmittel Ethanol, Isopropanol oder deren Mischungen ist.

## Claims

1. Cosmetic composition in the form of an aerosol lacquer for setting hair, characterised in that it contains, in a solvent, as film-forming substance, a tetrapolymer resulting from the copolymerisation:
   (a) of 5 to 10 % by weight of acrylic acid,
   (b) of 20 to 52 % by weight of N,N-dimethylacrylamide,
   (c) of 3 to 28 % by weight of N-tert-butylacrylamide, and
   (d) of 20 to 45 % by weight of ethyl methacrylate,
   the carboxylic acid functional groups of the said tetrapolymer being partially or completely neutralised with the aid of an inorganic or organic bases and, as propellent gas, at least one nonhalogenated compound.

2. Cosmetic composition according to Claim 1, characterised in that the tetrapolymer results from the copolymerisation:
   (a) of 5 to 8 % by weight of acrylic acid,
   (b) of 25 to 45 % by weight of N,N-dimethylacrylamide,
   (c) of 5 to 25 % by weight of N-tert-butylacrylamide, and
   (d) of 25 to 40 % by weight of ethyl methacrylate.

3. Cosmetic composition according to Claim 1 or 2, characterised in that the tetrapolymer has an absolute viscosity of between 2 and 5 mPa s (cP) measured in solution in DMF at a concentration of 5 % and at 34.6°C.

4. Cosmetic composition according to any one of the preceding claims, characterised in that the carboxylic acid functional groups of the tetrapolymer are neutralised in a proportion of at least 50 % and preferably of between 70 and 100 %.

5. Composition according to any one of Claims 1 to 4, characterised in that the carboxylic acid functional groups are neutralised with the aid of an inorganic base chosen from sodium hydroxide, potassium hydroxyde and aqueous ammonia.

6. Cosmetic composition according to any one of Claims 1 to 4, characterised in that the carboxylic acid functional groups are neutralised with the aid of an organic base chosen from triethanolamine, triisopropanolamine, 2-amino-2-methylpropanol and 2-amino-2-methyl-1,3-propanediol.

7. Cosmetic composition according to any one of the preceding claims, characterised in that the tetrapolymer is present in a concentration of between 0.5 and 5 % and preferably between 1 and 4 % by weight relative to the total weight of the composition.

8. Cosmetic composition according to any one of the preceding claims, characterised in that the propellent gas is present in a proportion of between 40 and 65 % by weight.

9. Cosmetic composition according to any one of the preceding claims, characterised in that the nonhalogenated propellent gas is a nonhalogenated hydrocarbon taken from the group consisting of propane, n-butane, isobutane and mixtures thereof.

10. Cosmetic composition according to Claim 9, characterised in that the nonhalogenated propellent gas is a propane-butanes mixture in a ratio of 1:10 to 1:2.

11. Cosmetic composition according to any one of Claims 1 to 9, characterised in that the nonhalogenated propellent gas is dimethyl ether or its mixture with at least one nonhalogenated hydrocarbon.

12. Cosmetic composition according to any one of the preceding claims, characterised in that the solvent is present in a proportion of between 40 and 60 % by weight relative to the total weight of the composition.

13. Cosmetic composition according to any one of the preceding claims, characterised in that the solvent is ethanol, isopropanol or mixtures thereof.